# EUROPÄISCHE PATENTSCHRIFT

(11) **EP 1 156 029 B1**
(45) Veröffentlichungstag und Bekanntmachung des Hinweises auf die Patenterteilung: **18.06.2003**
(21) Anmeldenummer: 01109289.7
(22) Anmeldetag: 17.04.2001
(51) Int. Cl.: C07C 69/716, C07C 67/347, C07C 45/67, C07C 49/12, C07C 45/66, C07C 49/587, C07C 45/62, C07C 49/385

(54) **Acetessigsäure-Derivate, Verfahren zu ihrer Herstellung und ihre Verwendung**
Derivatives of acetoacetic acid, their process of preparation and their use
Dérivés de l'acide acétoacétique,leur procédé de préparation et leur utilisation

(30) Priorität: 17.05.2000 DE 10023886
(43) Veröffentlichungstag der Anmeldung: 21.11.2001
(73) Patentinhaber: BASF AKTIENGESELLSCHAFT, 67056 Ludwigshafen (DE)
(72) Erfinder: Ebel, Klaus, Dr., 68623 Lampertheim (DE)

(56) Entgegenhaltungen:
- WO-A-00/01648
- US-A- 5 247 122
- J.C.ALLEN ET AL.: "Synthetic Aspects of Free-radical Addition. Part I. Radical-alkylation of Malonic Ester and Related Compounds" JOURNAL OF THE CHEMICAL SOCIETY., November 1962 (1962-11), Seiten 4468-4475, XP002174243 CHEMICAL SOCIETY. LETCHWORTH., GB

## Beschreibung

Die vorliegende Erfindung betrifft Acetessigsäure-Derivate der Formel Ia bzw. Ib, Verfahren zu ihrer Herstellung und ihre Verwendung zur Herstellung von 2,15-Hexadecandion. Ein weiterer Gegenstand der Erfindung ist die Verwendung der Verbindungen der Formel Ia bzw. Ib als Zwischenprodukt für die Herstellung von Muscon(3-Methyl-cyclopentadecanon).

Muscon(3-Methyl-cyclopentadecanon) der Formel III 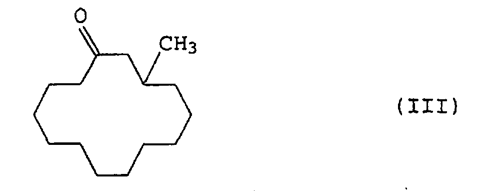 ist einer der wichtigsten Inhaltsstoffe der in der Parfümerie sehr begehrten natürlichen Moschusextrakte. Infolge des extrem hohen Preises für natürliche Extrakte ist die synthetische Herstellung von III von großem Interesse, insbesondere, da III allen anderen bekannten Moschusriechstoffen, wie den Tetralinoder Nitromoschusverbindungen, weit überlegen ist.

Die bisher angewendeten Herstellungsmethoden basieren überwiegend auf Ringerweiterungsreaktionen, ausgehend von Cyclododekanon (vgl. beispielsweise Helv. Chim. Acta 71 (1988), S. 1704-1718, und in loc. Cit. genannte Literatur). Diese Methoden weisen alle teilweise recht aufwendige mehrstufige Verfahrensschritt auf und sind daher für eine wirtschaftliche Nutzung unattraktiv.

Andere bekannte Synthesemethoden beinhalten intramolekulare Kondensationsreaktionen wie Aldol-, Dieckmann- oder Acyloinkondensation (siehe dazu Houben-Weyl, Methoden der organischen Chemie, Bd. 4/2, S. 729-815). Diese Methoden haben alle den großen Nachteil, daß nur in sehr hoher Verdünnung relativ gute Ausbeuten an Makrocyclen erhalten werden.

In Helv. Chim. Acta, 62 (1979), Seiten 2673-2680, wird ein neuartiges Syntheseverfahren für Muscon auf Basis 4,8-Dodecadiendiol vorgestellt. Schlüsselschritt ist hierbei die säurekatalysierte intramolekulare Cyclisierung eines offenkettigen Hydroxyacetals zum bicyclischen Dihydropyran, wobei allerdings wegen des notwendigen Verdünnungsprinzips hohe Lösungsmittelmengen erforderlich sind, wodurch dieses Verfahren nur für die Synthese von Labormengen sinnvoll einsetzbar ist.

Eine an sich recht gute Möglichkeit für die Herstellung von III schien die erstmals von Stoll (vgl. Helv. Chim. Acta, 30 (1974), Seiten 2019-2023) beschriebene Aldolkondensation ausgehend von Hexadecan-2,15-dion.

Allerdings war dieses Verfahren mit beachtlichen Nachteilen behaftet:
1) Waren die Herstellmöglichkeiten für das als Ausgangsmaterial benötigte 2,15-Diketon bislang unbefriedigend.
2) Sind die bei der Aldolkondensation zu erzielenden Ausbeuten trotz des Arbeitens in stark verdünnten Lösungen relativ gering (gemäß loc. cit. 17 %).

Nachteilig an dieser Synthese ist insbesondere die Verwendung des kostspieligen und außerdem toxikologisch bedenklichen 1,10-Dibromdecans.

Ferner wurde in J. Am. Chem. Soc. 82 (1960), Seiten 1447-1450, die folgende Synthese ausgehend von 2,2',5',2''-Terthienyl vorgestellt.

Zur Synthese größerer Diketonmengen ist diese Synthese jedoch wegen der schlechten Zugänglichkeit des Ausgangsmaterials nicht geeignet.

Zwei weitere Verfahren zur Herstellung des Diketons jeweils ausgehend von Butadien wurden von Tsuji et al. beschrieben:
a) in Chem. Lett. 1976, Seiten 773-774 und
b) in Bull. Chem. Soc. Japan, 51 (1978), Seite 1915.

In beiden Verfahren kommen teure Palladiumkatalysatoren zum Einsatz, wodurch auch diese Synthesen für einen industriellen Einsatz unattraktiv werden.

Weiterhin ist aus Bull. Chem. Soc. Japan, 56 (1983), Seiten 345-346, ein Verfahren zur Herstellung von IIa ausgehend von α,ω-Tetradecandicarbonsäure bekannt. Nachteilig an diesem Verfahren ist die schlechte Zugänglichkeit der Ausgangsverbindung.

Aus. J. Organomet. Chem. 264 (1984), Seiten 229-237, ist außerdem ein Verfahren zur Herstellung von IIa ausgehend von (CH₃)₃Si-CH₂-CH=CH-CH₂-CH₂-C(CH₃)=CH-CH₂-Si(CH₃)₃ bekannt. Nachteilig an diesem Verfahren ist neben der schlechten Zugänglichkeit der Ausgangsverbindung die Notwendigkeit der Verwendung von problematischen Reagentien, wie dem leicht entzündlichen Kaliumhydrid.

Weiterhin bekannt ist die in EP-B 0400509 offenbarte aber sehr aufwendige Herstellung von 2,15-Hexadecandion, die unter anderem Schritte wie eine Oxidation eines Diols mit Natriumhypochlorit zum Dialdehyd und entweder eine Wittig-Reaktion oder eine Grignard-Reaktion enthält.

Der vorliegenden Erfindung lag daher die Aufgabe zugrunde, einen einfachen und wirtschaftlichen Weg zur Herstellung von 2,15-Hexadecandion und damit auch zur Herstellung von Muscon zu finden, ohne die im Stand der Technik genannten Nachteile.

Die Aufgabe wurde erfindungsgemäß gelöst durch ein Verfahren zur Herstellung von Verbindungen der Formel Ia bzw. Ib 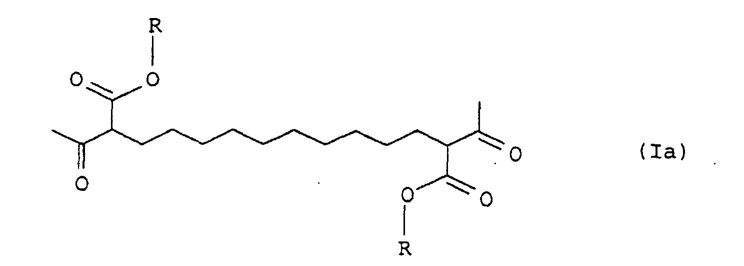 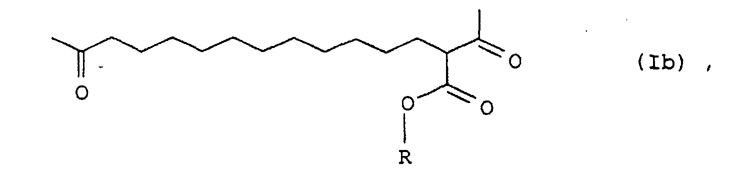 wobei R einen gegebenenfalls substituierten geradkettigen oder verzweigten C₁-C₆-Alkyl-, C₁-C₆-Alkoxyalkyl- oder einen Phenylrest bedeutet.
dadurch gekennzeichnet, daß man einen Acetessigsäureester der Formel II 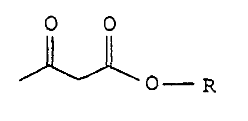 radikalisch an 1,9-Decandien anlagert und die so synthetisierten Verbindungen der Formel Ia bzw. Ib verseift und decarboxyliert (Schema 1)

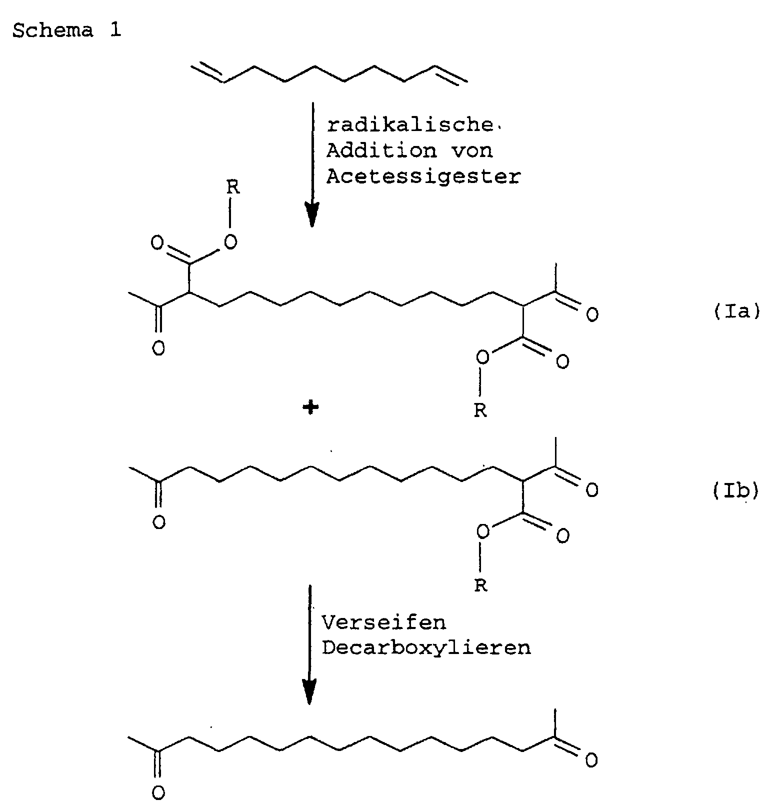

Dabei werden an das durch Metathese von Cycloocten mit Ethylen sehr einfach und kostengünstig herstellbare 1,9-Decadien zunächst in einer radikalischen Reaktion zwei Moleküle eines Esters der Acetessigsäure addiert.

Die so erhaltenen Additionsprodukte sind die entsprechenden Ester der 2,13-Bis-acetyl-1,14-tetradecandisäure (Ia). Daneben kann schon während der radikalischen Addition auch der entsprechende Ester der 2-Acetyl-pentdecan-14-on-säure (Ib) gebildet werden. Diese Verbindung entsteht durch Decarboxylierung einer Estergruppe des Primärproduktes.

Die genannten Verbindungen können auf die für Beta-Ketoester übliche Weise zum 2,15-Hexadecandion decarboxyliert werden.

Aus den Beta-Ketoestern können auch die entsprechenden Beta-Ketosäuren oder Salze der Säuren hergestellt werden, die beide als Zwischenprodukte bei Abspaltung der Estergruppen auftreten können. Die Beta-Ketosäuren sind allerdings keine stabilen Verbindungen, da sie beim Erwärmen sehr leicht zum 1,15-Hexadecandion decarboxylieren. Sie werden deshalb bevorzugt in Form ihrer Metallsalze isoliert.

Alkyl bei dem Rest R bedeutet, wenn nichts anderes angegeben ist, für sich oder in Kombination mit Alkoxy einen geradkettigen, verzweigten, gesättigten oder ungesättigten Rest mit 1 bis 6 Kohlenstoffatomen wie z.B. den Methyl-, Ethyl-, Propyl-, Isopropyl-, tert.-Butyl-, tert.-Pentyl-, Allyl -oder Propinylrest.

Vorzugsweise bedeutet Alkyl den Methyl-, Ethyl-, Propyl-, tert.-Butyl- oder tert.-Pentylrest.

Alkoxygruppen bedeuten eine Kombination einer Alkylgruppe gemäß der obigen Definition mit einem Sauerstoffatom, z.B. Methoxy-, Ethoxy-, Propoxy-, Butoxy- oder Pentoxygruppen. Besonders bevorzugt ist der 2-Methoxyethylrest.

Somit resultiert ein einfaches zweistufiges Verfahren zur Herstellung von 2,15-Hexadecandion, welches einen deutlichen Fortschritt gegenüber dem Stand der Technik bedeutet.

Da sich die 2,15-Diketone der allgemeinen Formel Ia bzw. Ib nach dem oben beschriebenen Verfahren in technisch einfacher Weise herstellen lassen, ergibt sich somit ein technisch einfacher und vorteilhafter Syntheseweg zu dem begehrten Moschusriechstoff Muscon.

Ein Weg der anschließenden intramolekularen Aldolkondensation des erfindungsgemäß hergestellten 2,15-Hexadecandions zu Muscon ist beispielsweise in (EP-B 0400509) offenbart.

Die nachfolgenden Beispiele sollen die Erfindung näher erläutern, ohne sie jedoch darauf zu beschränken.

### Beispiele

### Beispiel 1

### Herstellung von 2,13-Bis-acetyl-1,14-tetradecandisäuredimethylester und 2-Acetylpentdecan-14-on-säuremethylester

Zu 174 g (1,5 mol) Acetessigsäuremethylester tropft man unter Rühren bei 160°C innerhalb von 2 h ein Gemisch aus 116 g (1 mol) Acetessigsäuremethylester, 36 g (0,25 mol) 96 %iges 1,9-Decadien und 14,6 g (0,1 mol) Di-tert.-butylperoxid zu. Dann wird noch 1 h bei 160°C nachgerührt, um das Peroxid vollständig zu zersetzen. Schließlich wird der überschüssige Acetessigsäuremethylester im Vakuum abdestilliert. Der Rückstand von 82 g enthält nach GC-Analyse 57 % 2,13-Bis-acetyl-1,14-tetradecandisäuredimethylester (46,7 g = 0,14 mol) und 4 % 2-Acetyl-pentdecan-14-on-säuremethylester (3,3 g = 0,01 mol). Das entspricht einer Gesamtausbeute an beiden Produkten von 0,15 mol = 60 % Ausbeute.

Die reinen Verbindungen können durch Säulenchromatographie an Kieselgel (Laufmittel: Toluol/Aceton 4:1) gewonnen werden.
¹H-NMR-Daten (CDCl₃):
2,13-Bis-acetyl-1,14-tetradecandisäuredimethylester:
δ (ppm) = 1,3 (s, 16H), 1,8 (m, 4H), 2,2 (s, 6H), 3,4 (t, 2H), 3,7 (s, 6H)
2-Acetyl-pentdecan-14-on-säuremethylester:
δ (ppm) = 1,3 (s, 18H), 1,8 (m, 2H), 2,2 (s, 6H), 2,5 (t, 2H), 3,4 (t, 1H), 3,7 (s, 3H)

### Beispiel 2

### Herstellung von 2,15-Hexadecandion aus dem Produkt aus Beispiel 1

0,37 g (1 mmol), 2,13-Bis-acetyl-1,14-tetradecandisäuredimethylester wurden in 20 ml DMSO gelöst und mit 0,06 g (0,1 mmol) Natriumchlorid und 0,04 g (2 mmol) Wasser 3 h bei 155 bis 160°C am Rückfluß gekocht. Zur Aufarbeitung wird zunächst das DMSO im Vakuum abdestilliert und der Rückstand am Kugelrohr destilliert. Man erhält 0,21 g Hexadecan-1,15-dion. Danach beträgt die Ausbeute an 1,15-Hexadecandion bezogen auf 1,9-Decadien 82 %.

### Beispiel 3

### Herstellung von 2,13-Bis-acetal-1,14-tetradecandisäurediethylester und 2-Acetyl-pentdecan-14-on-säureethylester

Zu 325 g (2,5 mol) Acetessigsäureethylester tropft man unter Rühren bei 160°C innerhalb von 2 h aus getrennten Tropftrichtern gleichzeitig 36 g (0,25 mol) 95 %iges 1,9-Decadien und 14,6 g (0,1 mol) Di-tert.-butylperoxid zu. Dabei sinkt die Temperatur langsam auf 150°C und die Lösung beginnt zu sieden. Dann wird noch 1 h bei 150°C am Rückfluß gekocht, um das Peroxid vollständig zu zersetzen. Schließlich wird der überschüssige Acetessigsäuremethylester im Vakuum abdestilliert. Der Rückstand von 196 g enthält nach GC-Analyse 27 % 2,13-Bis-acetyl-1,14-tetradecandisäurediethylester (53 g = 0,14 mol) und 2 % 2-Acetyl-pentdecan-14-on-säureethylester (3,9 g = 0,01 mol). Das entspricht einer Gesamtausbeute an beiden Produkten von 0,15 mol = 60 % Ausbeute.

Die reinen Verbindungen können durch Säulenchromatographie an Kieselgel (Laufmittel: n-Hexan/Aceton 4:1) gewonnen werden.
¹H-NMR-Daten (CDCl₃) :
2,13-Bis-acetyl-1,14-tetradecandisäurediethylester:
δ (ppm) = 1,3 (m (t), 22H), 1,8, (m, 4H), 2,2 (s, 6H),
3,4 (t, 2H), 4,2 (q, 4H)
2-Acetyl-pentdecan-14-on-säureethylester:
δ (ppm) = 1,3 (m (t), 21H), 1,8 (m, 2H), 2,2 (s, 6H), 2,5 (t, 2H), 3,4 (t, 1H), 4,2 (q, 2H)

### Beispiel 5

### 2,13-Bis-acetyl-1,14-tetradecandisäure und ihr Natriumsalz

Man kocht Lösung von 0,37 g (1 mmol) 2,13-Bis-acetyl-1,14-tetradecandisäuredimethylester und 0,18 g (2,2 mmol) 50 %ige Natronlauge in 50 ml Methanol 18 h am Rückfluß. Dann wird das Methanol im Vakuum abdestilliert. Der Rückstand (0,45 g) enthält das gewünschte Natriumsalz.

Die freie Säure wird durch vorsichtiges Neutralisieren des Natriumsalzes mit Salzsäure erhalten. Sie decarboxyliert sehr leicht über die 2-Acetyl-pentdecan-14-on-säure als Zwischenprodukt zum 1,15-Hexadecandion und wurde nicht in reiner Form isoliert.

### Beispiel 6

### Herstellung von Hexadecan-1,15-dion ohne Isolierung der Zwischenprodukte

Zu 464 g (4 mol) Acetessigsäuremethylester tropft man bei 160°C aus getrennten Tropftrichtern (getaucht) innerhalb von 2 h 57,6 g (0,4 mol) 96 %iges 1,9-Decadien und 23,4 g (0,16 mol) Di-tert.butylperoxid zu und kocht anschließend noch 1 h am Rückfluß. Dann wird der überschüssige Acetessigester im Vakuum abdestilliert. Man erhält 148 g eines öligen Rückstandes, der direkt weiter umgesetzt wird. Dieser Rückstand wird in 400 ml DMSO gelöst und mit 35,6 g (0,44 mol) Natriumchlorid und 14,4 g (0,08 mol) Wasser 3 h bei 155 bis 160°C am Rückfluß gekocht. Nach GC-Analyse enthält die Mischung 10,7 Fl.-% Hexadecan-1,15-dion und noch 1,5 Fl.-% 2-Acetyl-pentdecan-14-on-säuremethylester. Zur Aufarbeitung wird zunächst das DMSO im Vakuum abdestilliert, der Rückstand (137 g) zwischen 700 ml Essigester und 100 ml Wasser verteilt und die Essigesterphase nochmals mit 100 ml Wasser gewaschen. Die Essigester-Phase wird getrocknet und im Vakuum eingeengt. Man erhält 102 g Rückstand mit einem Gehalt nach GC von 37 Gew.-% Hexadecan-1,15-dion. Danach beträgt die Ausbeute an 1,15-Hexadecandion bezogen auf 1,9-Decadien 42 %.

Der Rückstand wird im Hochvakuum destilliert, wobei 48 g 74%iges 1,15-Hexdecandion übergehen, was einer Ausbeute von 36 % entspricht.

## Patentansprüche

1. Verfahren zur Herstellung von Estern der Formel Ia und Ib 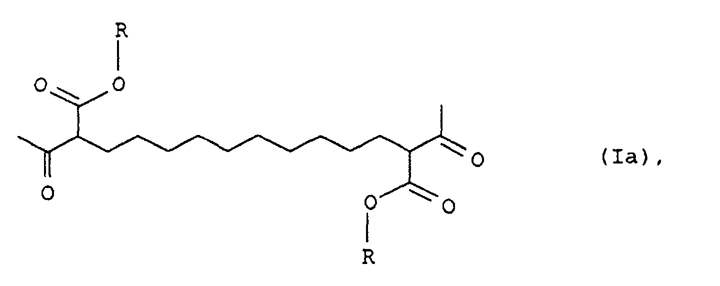 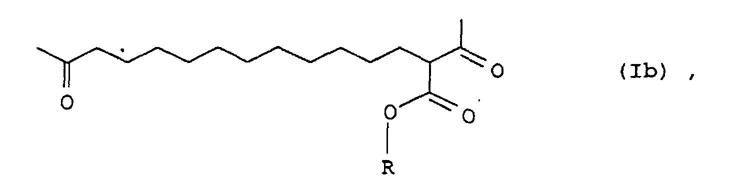 wobei R einen gegebenenfalls substituierten geradkettigen oder verzweigten C₁-C₆-Alkyl-, C₁-C₆-Alkoxyalkyl oder Phenylrest bedeutet,
**dadurch gekennzeichnet, daß** man einen Acetessigsäureester der Formel II 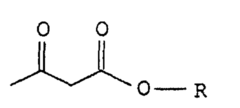 radikalisch an 1,9-Decandien anlagert.

2. Verfahren zur Herstellung eines Gemisches der Ester der Formeln Ia und Ib gemäß Anspruch 1.

3. Verbindungen der allgemeinen Formeln Ia und Ib 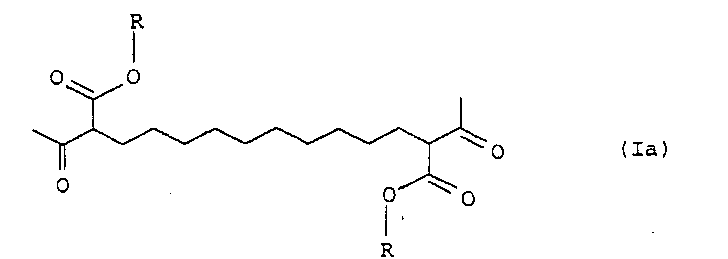 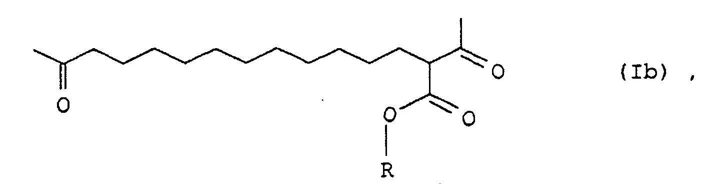 mit R = einen gegebenenfalls substituierten geradkettigen oder verzweigten C₁-C₆-Alkyl-, C₁-C₆-Alkoxyalkyl oder Phenylrest bedeutet,
Alkoxygruppen bedeuten eine Kombination einer Alkylgruppe gemäß der obigen Definition mit einem Sauerstoffatom, z.B. Methoxy-, Ethoxy-, Propoxy-, Butoxy- oder Pentoxygruppen, deren Ester, optisch aktive Formen, Racemate, Tautomere, Diasteromerengemische und Salze davon.

4. Verfahren zur Herstellung von 1,15-Hexandecandion, **dadurch gekennzeichnet, daß** man die Ester der Acetessigsäure der Formel II radikalisch an 1,9-Hexadien addiert und anschließend die Verbindungen der Formel Ia bzw. Ib zum 1,15-Hexandecandion decarboxyliert.

5. Verfahren zur Herstellung von Muscon der Formel III, 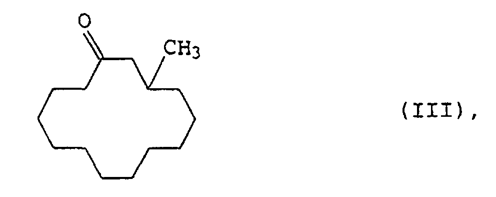 **dadurch gekennzeichnet, daß** man Ester der Acetessigsäure (II) an 1,9-Decandien anlagert, die Anlagerungsprodukte Ia bzw. Ib zum 1,15-Hexadecandion decarboxyliert, dieses anschließend in der Gasphase zu Muscon im Gemisch mit ungesättigten Muscon-Derivaten cyclisiert und schließlich die ungesättigten Muscon-Derivate zum Muscon hydriert.

6. Verwendung der Verbindungen der allgemeinen Formel Ia bzw. Ib 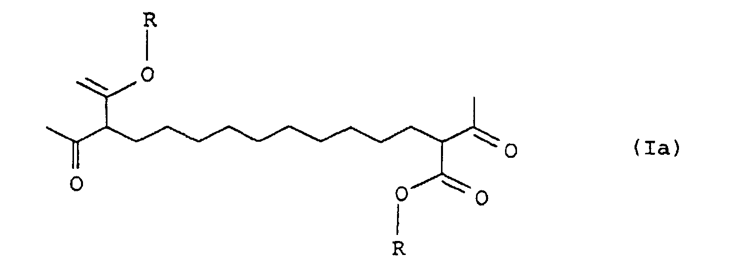 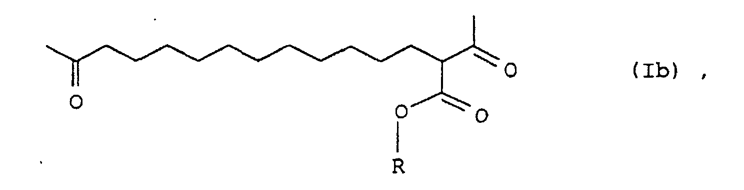 zur Herstellung von 2,15-Hexadecandion.

7. Verwendung der Verbindungen der allgemeinen Formel Ia bzw. Ib als Zwischenprodukte zur Herstellung von Muscon der Formel III

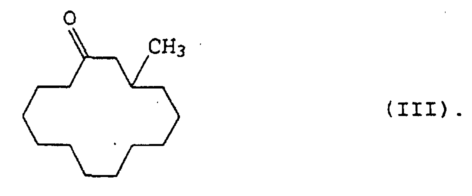

## Claims

1. A process for the preparation of esters of the formula Ia and Ib 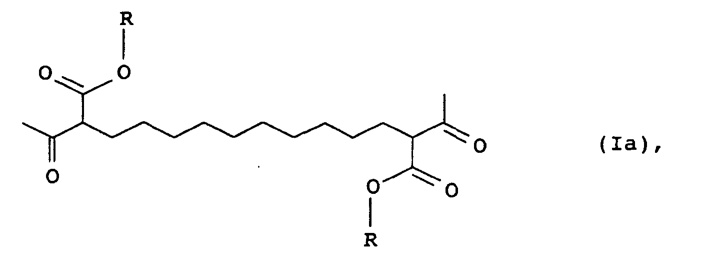 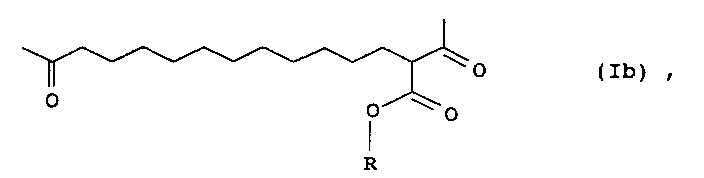 where R is an optionally substituted straight-chain or branched C₁-C₆-alkyl, C₁-C₆-alkoxyalkyl or phenyl radical, which comprises adding an acetoacetic ester of the formula II 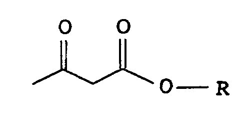 to 1,9-decadiene by a free-radical means.

2. A process for the preparation of a mixture of the esters of the formulae Ia and Ib as claimed in claim 1.

3. A compound of the formula Ia or Ib 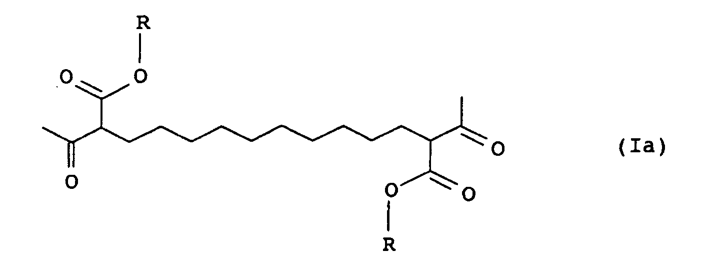 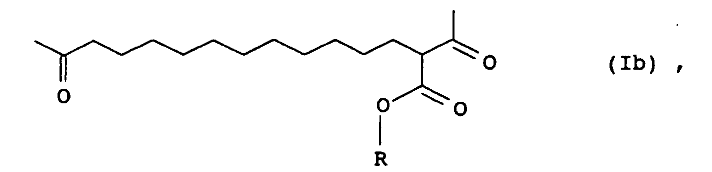 where R = an optionally substituted straight-chain or branched C₁-C₆-alkyl, C₁-C₆-alkoxyalkyl or phenyl radical, alkoxy groups are a combination of an alkyl group according to the above definition with an oxygen atom, e.g. methoxy, ethoxy, propoxy, butoxy or pentoxy groups, ester thereof, optically active form, racemate, tautomer, diastereomer mixture and salt thereof.

4. A process for the preparation of 1,15-hexadecanedione, which comprises adding the esters of acetoacetic acid of the formula II to 1,9-hexadiene by free-radical means and then decarboxylating the compounds of the formula Ia or Ib to give 1,15-hexadecanedione.

5. A process for the preparation of muscone of the formula III 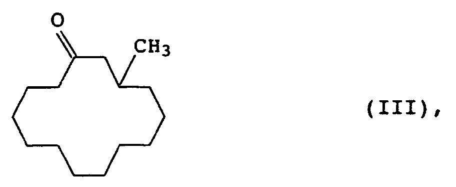 which comprises adding esters of acetoacetic acid (II) to 1,9-decadiene, decarboxylating the addition products Ia or Ib to give 1,15-hexadecanedione, then cyclizing the latter in the gaseous phase to give muscone in admixture with unsaturated muscone derivatives, and finally hydrogenating the unsaturated muscone derivatives to give muscone.

6. The use of the compounds of the formula Ia or Ib 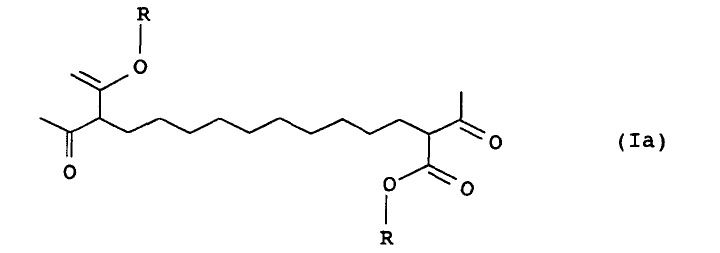 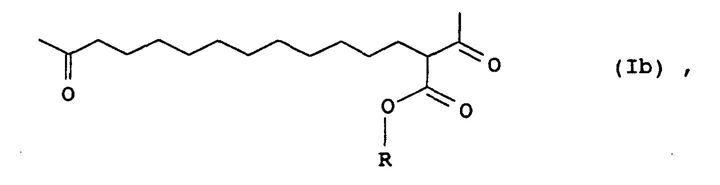 for the preparation of 2,15-hexadecanedione.

7. The use of the compounds of the formula Ia or Ib as intermediates for the preparation of muscone of the formula III

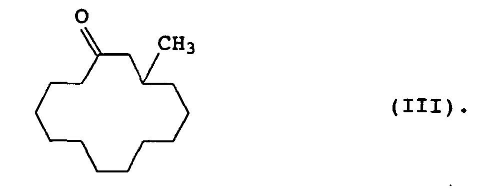

## Revendications

1. Procédé pour la préparation d'esters de formule Ia et Ib 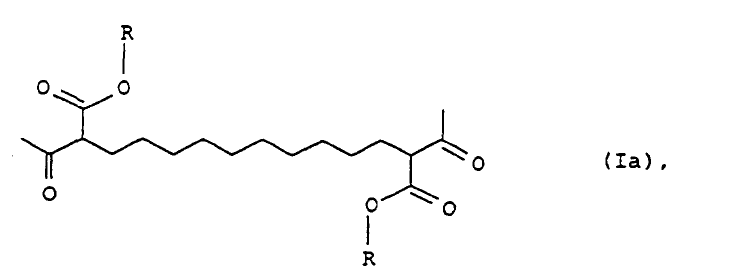 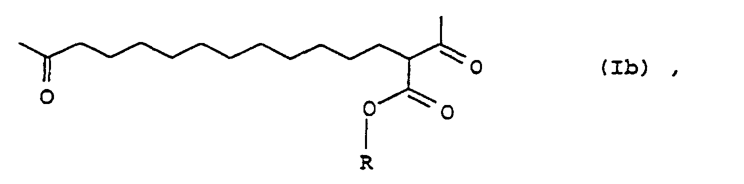 où R représente un reste alkyle en C₁-C₆ ou alcoxyalkyle en C₁-C₆ ou phényle, linéaire ou ramifié, éventuellement substitué,
**caractérisé par le fait qu'**on fixe par addition un ester d'acide acétoacétique de formule II 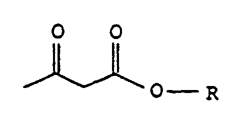 par voie radicalaire sur du 1,9-décanediène.

2. Procédé pour la préparation d'un mélange d'esters de formules Ia et Ib selon la revendication 1.

3. Composés de formules générales Ia et Ib 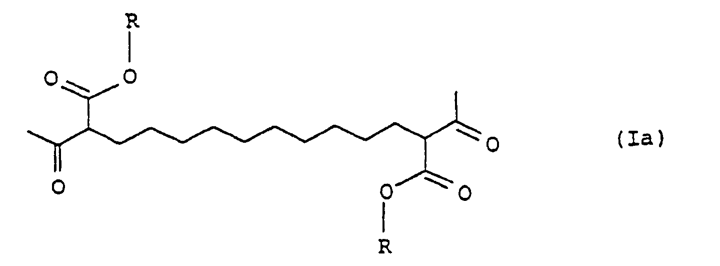 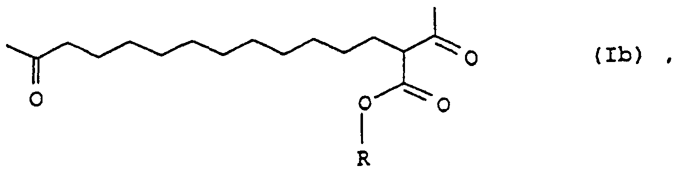 avec R désignant un groupe alkyle en C₁-C₆, alcoxyalkyle en C₁-C₆ ou phényle, linéaire ou ramifié, éventuellement substitué,
tandis que les groupes alcoxy représentent une combinaison d'un groupe alkyle selon la définition susdite avec un atome d'oxygène, par exemple des groupes méthoxy, éthoxy, propoxy, butoxy ou pentoxy, leurs esters, formes optiquement actives, racémiques tautomères, mélanges de diastéréoisomères et sels.

4. Procédé pour la préparation de 1,15-hexadécanedione, **caractérisé par le fait qu'**on ajoute les esters de l'acide acétoacétique de formule II par voie radicalaire à du 1,9-hexadiène et on décarboxyle ensuite les composés de formule Ia ou Ib pour donner la 1,15-hexadécanedione.

5. Procédé pour la préparation de muscone de formule III 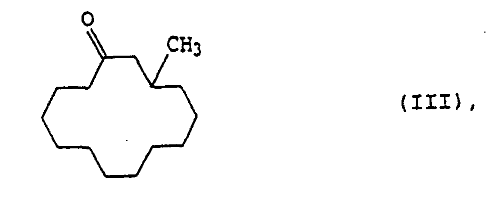 **caractérisé par le fait qu'**on fixe par addition de l'ester d'acide acétoacétique (II) sur du 1,9-décanediène, on décarboxyle les produits d'addition Ia ou Ib pour former la 1,15-hexadécanedione, on cyclise ensuite celle-ci en phase gazeuse pour former la muscone en mélange avec des dérivés insaturés de muscone, et on hydrogène finalement les dérivés insaturés de muscone pour donner la muscone.

6. Utilisation des composés de formule générale la ou Ib 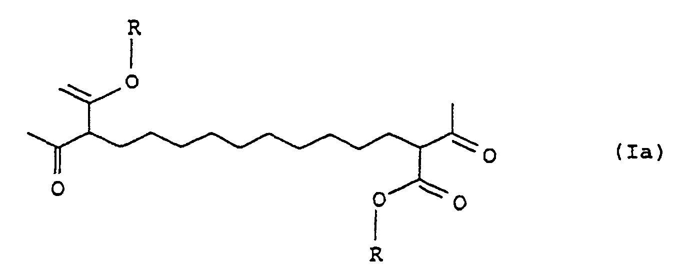 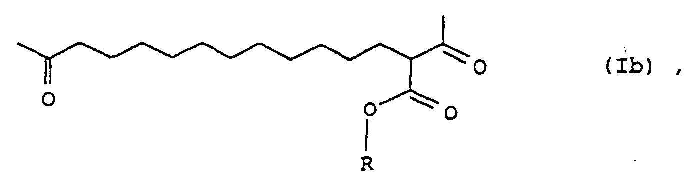 pour la préparation de 2,15-hexadécanedione.

7. Utilisation des composés de formule générale Ia ou Ib comme produits intermédiaires pour la préparation de muscone de formule III
